# EUROPEAN PATENT APPLICATION

(11) **EP 4 167 246 A1**
(43) Date of publication of application: **19.04.2023**
(21) Application number: 21842067.7
(22) Date of filing: 15.07.2021
(51) Int. Cl.: G16H 50/20, G06Q 30/02

(54) **HEADACHE CAUSE DETERMINATION SYSTEM, HEADACHE CAUSE DETERMINATION METHOD, PROGRAM, AND DATA STRUCTURE**

(30) Priority: 17.07.2020 JP 2020123104
(71) Applicant: Mitsui Chemicals, Inc., Tokyo 105-7122 (JP)
(72) Inventor: KOBAYASHI, Seiichi, Tokyo 105-7117 (JP); TAKEUCHI, Yoshito, Tokyo 105-7117 (JP)
(74) Representative: Hoffmann Eitle
(86) International application number: PCT/JP2021/026681
(87) International publication number: WO 2022/014689

(57) **Abstract**

A headache cause determination system that is configured to determine whether or not there is a possibility that a cause of a migraine symptom, of a user suffering from the migraine symptom, is a light-based cause, the system comprising a computer. The computer includes a CPU, a storage unit, and a determination unit executed by the CPU. The storage unit is configured to store data that enables a determination as to whether or not there is a possibility that a cause of the migraine symptom of the user is a light-based cause, based on question information regarding the migraine symptom of the user and response information regarding the user's response to the question information. The determination unit is configured to determine whether or not there is a possibility that a cause of the migraine symptom of the user is a light-based cause, based on the response information regarding the user's response to the question information and based on the data stored in the storage unit, and to output a result of the determination.

## Description

### Technical Field

The present disclosure relates to a headache cause determination system, a headache cause determination method, a program, and a data structure.

### Background Art

Japanese Patent Application Laid-Open (JP-A) No. 2000-325840 discloses a conventional technique related to a system that colors a lens based on coloring information on coloring the lens.

### SUMMARY OF INVENTION

### Technical Problem

It is desirable that the appropriate material, hue, coating type, and the like of a lens be selected according to the way a user wears and uses eyeglasses in which the lens is incorporated and to the user's physical condition. For example, there are individuals who have various headache symptoms and some of them suffer from a migraine symptom. It is preferable that a lens effective for alleviating migraine be selected as the lens of eyeglasses for individuals suffering from migraine to wear. Migraine is reported by WHO as one of diseases that shorten people's healthy life. It can be said that lens processing that accommodates the needs of users suffering from such a disease is a social demand to be met to extend the healthy life.

Migraine is triggered by various causes. The causes are complex in some cases, and therefore identifying a specific cause of the disease is difficult. In a case where a cause of migraine is identified as light, a system that supports or proposes selection of a lens dealing with migraine is required.

An object of the present disclosure is to provide a headache cause determination system, a program, and a data structure that can determine whether a cause of a migraine symptom of a user is light while taking into consideration answers to a plurality of questions.

### Solution to Problem

A headache cause determination system that is configured to determine whether or not there is a possibility that a cause of a migraine symptom, of a user suffering from the migraine symptom, is a light-based cause, the system comprising a computer, wherein: the computer includes a CPU, a storage unit, and a determination unit executed by the CPU,
the storage unit is configured to store data that enables a determination as to whether or not there is a possibility that a cause of the migraine symptom of the user is a light-based cause, based on question information regarding the migraine symptom of the user and response information regarding the user's response to the question information, and the determination unit is configured to determine whether or not there is a possibility that a cause of the migraine symptom of the user is a light-based cause, based on the response information regarding the user's response to the question information and based on the data stored in the storage unit, and to output a result of the determination.

In the headache cause determination system of the present disclosure, wherein: when the determination unit has determined that there is a possibility that a cause of a migraine symptom of the user is a light-based cause, the storage unit further stores lens information regarding an ophthalmic lens recommended as a lens to be used for preventing or alleviating the migraine symptom, and when the determination unit has determined that there is a possibility that a cause of the migraine symptom of the user is a light-based cause, the determination unit outputs a result of the determination, and, from the lens information, selects and outputs the ophthalmic lens recommended as the lens to be used for preventing or alleviating the migraine symptom of the user.

In the headache cause determination system of the present disclosure, the storage unit further stores user information including information regarding a residence of the user, and retail store information regarding a retail store that sells an ophthalmic lens recommended as a lens to be used for preventing or alleviating a migraine symptom of the user, and when selecting and outputting an ophthalmic lens recommended as a lens to be used for preventing or alleviating the migraine symptom of the user, the determination unit selects and outputs a candidate for a retail store that sells the ophthalmic lens recommended as the lens to be used for preventing or alleviating the migraine symptom of the user, based on the information regarding the residence of the user and the retail store information.

According to the headache cause determination system of the present disclosure, the question information comprises a question about a condition of the user who is developing the migraine symptom, a question about a cause that triggers the migraine symptom, or a question about a condition of the user before and after the user develops the migraine symptom.

According to the headache cause determination system of the present disclosure, the question about a condition of the user who is developing the migraine symptom is presented in order to ascertain whether light intensifies a migraine symptom of the user when the user is developing the migraine symptom or whether the user feels light to be dazzling or unpleasant when the user is developing the migraine symptom.

According to the headache cause determination system of the present disclosure, the question about a condition of the user who is developing the migraine symptom is presented in order to ascertain whether light causes the user any pain inside or surrounding their eyes when the user is developing migraine symptom.

According to the headache cause determination system of the present disclosure, the question about a cause that triggers the migraine symptom of the user is presented in order to ascertain whether the user has experienced a case in which light has triggered the migraine symptom.

According to the headache cause determination system of the present disclosure, the question about the condition of the user before and after the user develops the migraine symptom is presented in order to ascertain whether the user has experienced a case in which the user has become sensitive to light in a period between one migraine symptom subsiding and another migraine symptom starting.

A headache cause determination method according to the present disclosure is a headache cause determination method that causes a computer to execute processing, the processing comprising: determining whether or not there is a possibility that a cause of a migraine symptom of a user is a light-based cause, based on data that enables a determination as to whether or not there is a possibility that a cause of a migraine symptom of a user is a light-based cause, the determination being based on question information regarding the migraine symptom of the user and response information regarding the user's response to the question information, the data being stored in a storage unit of the computer; and outputting a result of the determination.

The headache cause determination method according to the present disclosure causes a computer to execute the processes of: causing the storage unit to further store user information including information regarding a residence of the user and retail store information regarding a retail store that sells an ophthalmic lens recommended as a lens to be used for preventing or alleviating a migraine symptom of the user; and when selecting and outputting an ophthalmic lens recommended as a lens to be used for preventing or alleviating the migraine symptom of the user, selecting and outputting a candidate for a retail store that sells the ophthalmic lens recommended as the lens to be used for preventing or alleviating the migraine symptom of the user, based on the information regarding the residence of the user and the retail store information.

According to the headache cause determination method of the present disclosure, the question information comprises a question about a condition of the user who is developing a migraine symptom, a question about a cause that triggers a migraine symptom of the user, or a question about a condition of the user before and after the user develops a migraine symptom.

A program according to the present disclosure is a program that causes a computer to function, in a headache cause determination system including a storage unit and a determination unit, is executable by a computer to function as the determination unit, the determination unit being configured to: determine whether or not there is a possibility that a cause of a migraine symptom of a user is a light-based cause, based on data that enables a determination as to whether or not there is a possibility that a cause of a migraine symptom of a user is a light-based cause, the determination being based on question information regarding the migraine symptom of the user and response information regarding the user's response to the question information, the data being stored in a storage unit of the computer; and output a result of the determination.

The program according to the present disclosure causes the storage unit to further store user information including information regarding a residence of the user and retail store information regarding a retail store that sells an ophthalmic lens recommended as a lens to be used for preventing or alleviating a migraine symptom of the user, and when the determination unit selects and outputs an ophthalmic lens recommended as a lens to be used for preventing or alleviating the migraine symptom of the user, the program causes the determination unit to select and output a candidate for a store that sells the ophthalmic lens recommended as the lens to be used for preventing or alleviating the migraine symptom of the user, based on the information regarding the residence of the user and the retail store information.

According to the program of the present disclosure, the question information comprises a question about a condition of the user who is developing a migraine symptom, a question about a cause that triggers a migraine symptom of the user, or a question about a condition of the user before and after the user develops a migraine symptom.

A data structure according to the present disclosure is a data structure including question information regarding a migraine symptom of a user; response information regarding the user's response to the question information; and data that enables a determination as to whether or not there is a possibility that a cause of the migraine symptom of the user is a light-based cause, based on the question information and the response information,
wherein the data structure causes a computer to determine whether or not there is a possibility that a cause of the migraine symptom of the user is a light-based cause, based on the response information regarding the user's response to the question information and on the data, and to output a result of the determination.

The data structure according to the present disclosure further includes user information including information regarding a residence of the user; and retail store information regarding a retail store that sells an ophthalmic lens recommended as a lens to be used for preventing or alleviating a migraine symptom of the user, wherein, when an ophthalmic lens recommended as a lens to be used for preventing or alleviating the migraine symptom of the user has been selected and output, the data structure causes a computer to select and output a candidate for a store that sells the ophthalmic lens recommended as the lens to be used for preventing or alleviating the migraine symptom of the user, based on the information regarding the residence of the user and the retail store information.

According to the data structure of the present disclosure, the question information comprises a question about a condition of the user who is developing a migraine symptom, a question about a cause that triggers a migraine symptom of the user, or a question about a condition of the user before and after the user develops a migraine symptom.

### Advantageous Effects of Invention

The headache cause determination system, the headache cause determination method, the program, and the data structure of the present disclosure offer an effect that whether a cause of a migraine symptom of a user is light can be determined as answers to a plurality of questions are taken into consideration.

### BRIEF DESCRIPTION OF DRAWINGS

Fig. 1 is a block diagram showing a system configuration of a headache cause determination system according to a first embodiment.
Fig. 2 is a schematic block diagram of a computer functioning as a headache cause determination apparatus according to the first embodiment.
Fig. 3 depicts an example of question information and answer information according to the first embodiment.
Fig. 4A depicts an example of a screen of an input interface of a terminal.
Fig. 4B depicts an example of a screen of the input interface of the terminal.
Fig. 4C depicts an example of a screen of the input interface of the terminal.
Fig. 4D depicts an example of a screen of the input interface of the terminal.
Fig. 4E depicts an example of a screen of the input interface of the terminal.
Fig. 4F depicts an example of a screen of the input interface of the terminal.
Fig. 4G depicts an example of a screen of the input interface of the terminal.
Fig. 4H depicts an example of a screen of the input interface of the terminal.
Fig. 5 depicts an example of a process routine executed by the headache cause determination apparatus according to the first embodiment.
Fig. 6A depicts an example of question information and answer information according to a second embodiment.
Fig. 6B depicts an example of the question information and the answer information according to the second embodiment.
Fig. 7A depicts an example of question information and answer information according to a third embodiment.
Fig. 7B depicts an example of the question information and the answer information according to the third embodiment.
Fig. 8 is a block diagram showing a system configuration of a headache cause determination apparatus according to a fourth embodiment.

### DESCRIPTION OF EMBODIMENTS

Embodiments of the present invention will hereinafter be described in detail. A headache cause determination apparatus 200 according to the embodiments determines and outputs a headache cause, based on answer information ("answer information" is also referred as "response information" in this specification) inputted. A user according to the embodiment is, for example, a customer who suffers from a migraine symptom and intends to purchase a lens at a store selling eyeglasses.

### <System Configuration According to First Embodiment>

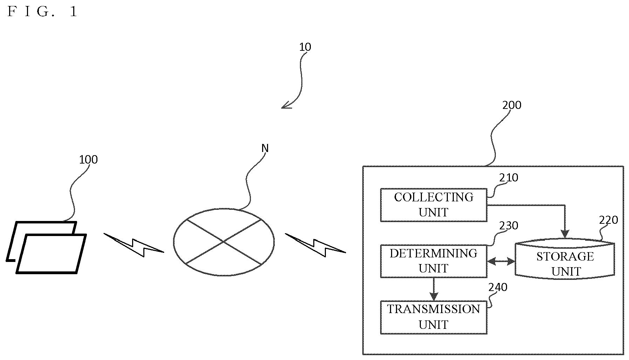

Fig. 1 is a block diagram showing a system configuration of a headache cause determination system 10 according to a first embodiment. As shown in Fig. 1, in the headache cause determination system 10, a plurality of terminals and the headache cause determination apparatus 200 are interconnected via a network N.

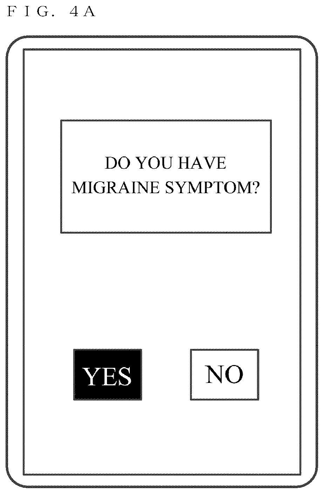

A terminal 100 shown in Fig. 1 is a terminal device, such as a smartphone a user owns or a tablet terminal assigned to a store selling eyeglasses. The terminal 100 has an interface, as shown in Figs. 4A to 4H, which will be referred to later. The interface is used to display question information on a migraine symptom of the user, input answer information on answers to the question information, and input user information including information on the residence of the user. The interface is used also to display information on an ophthalmic lens, the information being received from the headache cause determination apparatus 200, and store information on a store selling the ophthalmic lens. The terminal 100 transmits the answer information and user information, together with identification information assigned to the terminal 100, to the headache cause determination apparatus 200. The identification information is identification information on the terminal 100 and on the user having acquired the information.

The headache cause determination apparatus 200 having the configuration shown in Fig. 1 can be provided as a computer including a CPU, a RAM, and a ROM storing programs and various data for executing each of process routines which will be described later.

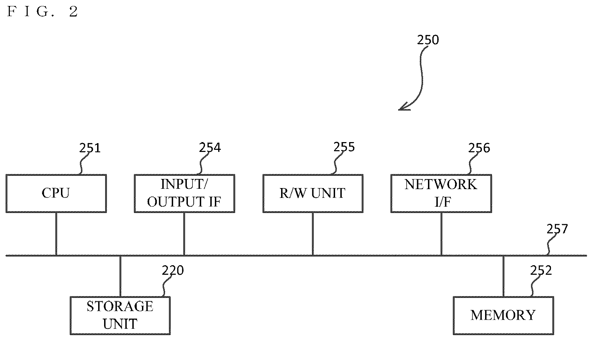

For example, the headache cause determination apparatus 200 can be provided as a computer 250 shown in Fig. 2. The computer 250 includes a CPU 251, a memory 252 serving as a temporary storage area, and a nonvolatile storage unit 220. The computer 250 further includes an input/output interface (I/F) 254 to which an input/output device or the like (not illustrated) is connected, and a read/write (R/W) unit 255 that controls data reading/writing from/to a recording medium. The computer 250 further includes a network I/F 256 connected to a network, such as the Internet. The CPU 251, the memory 252, the storage unit 220, the input/output I/F 254, the R/W unit 255, and the network I/F 256 are interconnected via a bus 257.

The storage unit 220 can be provided as a hard disk drive (HDD), a solid state drive (SSD), a flash memory, or the like. The storage unit 220, which serves as a storage medium, stores data that allows determining the presence or absence of a possibility of a cause of a migraine symptom of the user being a light-based cause and a program for causing the computer 250 to function. The CPU 251 reads a program from the storage unit 220, loads the program onto the memory 252, and sequentially executes processes included in the program.

The above description is the description of an example of an electrical configuration of the computer shown in Fig. 2.

The storage unit 220 stores question information, answer information, data, user information on the user, lens information, and store information. The question information is information on a migraine symptom of the user. The answer information is information on answers to the question information inputted by the user. The data is a program that can determine the presence or absence of a possibility of a cause of a migraine symptom of the user being a light-based cause. The lens information is information on an ophthalmic lens recommended as a lens to be used for preventing or alleviating a migraine symptom. The store information is information on a store that sells the ophthalmic lens recommended as the lens to be used for preventing or alleviating the migraine symptom.

In one case, the entire answer information and user information are stored in the storage unit 220 after a determination unit 230, which will be described later, determines the presence or absence of the possibility of the cause of the migraine symptom of the user being the light-based cause. In other cases, only some pieces of answer information and of user information may be stored as the rest of answer information and of user information are deleted or stored as anonymous information. The answer information and user information may be stored in an encrypted form.

Information stored in the storage unit 220 includes information stored temporarily. In other words, answer information and user information inputted by the user are stored temporarily, and based on the stored information, the determination unit 230 determines the presence or absence of the possibility of the cause of the migraine symptom of the user being the light-based cause. Following the determination by the determination unit 230, the answer information and the user information used for the determination are deleted.

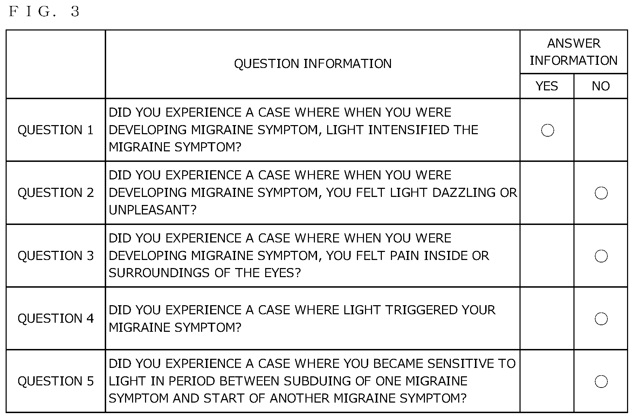

Question information on a migraine symptom of the user will be described with reference to Fig. 3.

The question information includes a question about a condition of the user who is developing a migraine symptom, a question about a cause that triggers a migraine symptom of the user, and a question about a condition of the user before and after the user's developing a migraine symptom.

The question about a condition of the user who is developing a migraine symptom is presented for the purpose of knowing whether light intensifies a migraine symptom of the user when the user is developing the migraine symptom or whether the user feels light dazzling or unpleasant when the user is developing a migraine symptom.

For example, such a question is included in the question information shown in Fig. 3, as a question 1 "Did you experienced a case where when you were developing a migraine symptom, light intensified the migraine symptom?" and "Did you experienced a case where when you were developing a migraine symptom, you felt light dazzling or unpleasant?".

The question about a condition of the user who is developing a migraine symptom further includes a question presented for the purpose of knowing whether light causes the user a pain inside or the surroundings of the eyes when the user is developing migraine symptom.

For example, such a question is included in the question information shown in Fig. 3, as a question 3 "Did you experienced a case where when you were developing a migraine symptom, you felt a pain inside or the surroundings of the eyes?".

The question about a cause that triggers a migraine symptom of the user is presented for the purpose of knowing whether the user experiences a case where light triggers the migraine symptom.

For example, such a question is included in the question information shown in Fig. 3, as a question 4 "Did you experienced a case where light triggered your migraine symptom?".

The question about a condition of the user before and after the user's developing a migraine symptom includes a question presented for the purpose of knowing whether the user experiences a case where the user becomes sensitive to light in a period between subduing of one migraine symptom and the start of another migraine symptom.

For example, such a question is included in the question information shown in Fig. 3, as a question 5 "Did you experience a case where you became sensitive to light in a period between subduing of one migraine symptom and the start of another migraine symptom?".

Questions making up the question information are not limited to a list of questions shown in Fig. 3. The question information may include other questions, and not necessarily include all the questions shown in Fig. 3.

Answer information, i.e., the user's answers to the questions making up the question information is entered in a table of Fig. 3. An answer to the question 1 is entered as "Yes" or "No". An answer to the question 2 is entered as "Yes" or "No". An answer to the question 3 is entered as "Yes" or "No". An answer to the question 4 is entered as "Yes" or "No". An answer to the question 5 is entered as "Yes" or "No". In the table of Fig. 3, either a "Yes" answer column or a "No" answer column is chosen to enter an answer "∘ (circle)" therein. Each answer column chosen is filled with "∘ (circle)" as an answer column not chosen is left blank. This table of Fig. 3, for example, shows an entry record that "Yes" answer is chosen for the question 1 only as "No" answer is chosen for the other questions 2 to 5.

Fig. 3 indicates that answer columns each filled with "∘ (circle)", the answer columns making up the answer information, are columns chosen by the user.

Data that allows determining the presence or absence of a possibility of a cause of a migraine symptom of the user being a light-based cause is, for example, a program that when at least one of answers to questions making up the question information shown in Fig. 3 is "Yes", determines the presence of a possibility of a cause of a migraine symptom of the user being a light-based cause. This program will be described later.

User information (not illustrated) includes information on the user's residence, information on the user's age, information on the user's gender, information on the user's occupation, and information on the user's living environment.

The user information, which is not limited to the above pieces of information, may further include additional information, e.g., a prescription from a doctor.

A customer database having a history of user information recorded therein may be provided. This customer database contains a record of answer information as well.

Lens information on an ophthalmic lens recommended as a lens to be used for preventing or alleviating a migraine symptom includes information (not illustrated) on an ophthalmic lens that selectively cuts light with a wavelength close to 480 nm.

It is a known fact that an ophthalmic lens that selectively cuts light with a wavelength close to 480 nm is effective for alleviating photosensitive migraine.

Specifically, the retina of mammals carries three cones, i.e., an S cone, an M cone, and an L cone, a rod, and intrinsically photosensitive retinal ganglion cells or ipRGC, which are photoreceptors discovered in recent years. The S cone, the M cone, the L cone, the rod, and the ipRGC have their sensitivity peaks at wavelengths of 420 nm, 530 nm, 560 nm, 500 nm, and 480 nm, respectively.

For example, given a fact that ipRGC strongly reacts to blue component light with a wavelength of around 480 nm, it is concluded that using eyewear, such as eyeglasses or sunglasses containing an optical material showing low transmittance against blue component light in the wavelength range of (2) as an optical material of the present disclosure dose, offers the user effects of preventing/alleviating light-sensitive migraine and preventing/alleviating asthenopia and the like.

In addition to information on an ophthalmic lens recommended as a lens to be used for preventing or alleviating a migraine symptom, the lens information stored in the storage unit 220 may further include information on an ophthalmic lens for digital device users that selectively cuts light with a wavelength close to 460 nm and an ophthalmic lens that cuts light with other wavelengths.

Store information on a store that sells an ophthalmic lens recommended as a lens to be used for preventing or alleviating a migraine symptom of the user includes the name, address, and contact information (telephone number, e-mail address, etc.) of the store (not illustrated).

Processing units of the headache cause determination apparatus 200 of Fig. 1 will hereinafter be described. As shown in Fig. 1, the headache cause determination apparatus 200 includes, in terms of functional configuration, a collecting unit 210, the storage unit 220, the determination unit 230, and a transmission unit 240.

The collecting unit 210 acquires incoming answer information and user information from the terminal. The collecting unit 210 may read global positioning system (GPS) information of the terminal when the user information includes no information on the residence of the user, and may read the GPS information even when the user information includes information on the residence of the user.

The determination unit 230 determines the presence or absence of a possibility of a cause of a migraine symptom of the user being a light-based cause, based on the answer information on the user's answers to the question information and on data stored in the storage unit 220, and outputs a result of the determination.

Specifically, when determining the presence of a possibility of a cause of a migraine symptom of the user being a light-based cause, the determination unit 230 outputs a result of that determination. Then, from the lens information stored in the storage unit 220, the determination unit 230 determines an ophthalmic lens that selectively cuts light with a wavelength close to 480 nm to be an ophthalmic lens recommended as a lens to be used for preventing or alleviating a migraine symptom of the user, and transmits a result of the determination to the terminal through the transmission unit 240.

In addition, when determining the ophthalmic lens recommended as the lens to be used for preventing or alleviating the migraine symptom of the user and outputting a result of the determination, the determination unit 230 determines a candidate for a store that sells the ophthalmic lens recommended as the lens to be used for preventing or alleviating the migraine symptom of the user, based on information on the residence of the user and the store information stored in the storage unit 220. The determination unit 230 outputs a result of the determination to the terminal 100 through the transmission unit 240. It is preferable that the determined store candidate be a store situated in close proximity to the residence of the user or an office the user works at.

The presence or absence of a possibility of a cause of a migraine symptom of the user being a light-based cause is determined such that the presence of a possibility of a cause of a migraine symptom of the user being a light-based cause is determined when at least one of answers to questions making up the question information shown in Fig. 3 is "Yes".

Determination on the presence or absence of a possibility of a cause of a migraine symptom of the user being a light-based cause is, however, not limited to the case where the presence of a possibility of a cause of a migraine symptom of the user being a light-based cause is determined when at least one of answers to questions making up the question information shown in Fig. 3 is "Yes". Each of the questions may be set as a weighted question. For example, with regard to the questions 1 to 3, a case may be adopted where the presence of a possibility of a cause of a migraine symptom of the user being a light-based cause is determined when at least one of answers to the questions 1 to 3 is "Yes". With regard to the questions 4 and 5, a case may be adopted where the presence of a possibility of a cause of a migraine symptom of the user being a light-based cause is determined when answers to the questions 4 and 5 are both "Yes". Another case may also be adopted where the presence of a possibility of a cause of a migraine symptom of the user being a light-based cause is determined only when one of answers to the questions 1 to 3 is "Yes" and either an answer to the question 4 or an answer to the question 5 is "Yes".

The transmission unit 240 transmits information on an ophthalmic lens and information on a candidate for a store that sells the ophthalmic lens, to the terminal 100 to which identification information is assigned, the information being transmitted as a result of a determination the determination unit 230 makes on the identification information.

Such information on the ophthalmic lens and information on a candidate for a store that sells the ophthalmic lens are displayed on the interface of the terminal 100.

It is preferable that candidates for stores that sell the ophthalmic lens be displayed, for example, in the order of the stores' proximity to the user's residence.

Next, examples of screens of the interface of the terminal will then be described with reference to Figs. 4A to 4H. As shown in Figs. 4A to 4H, the interface of the terminal is a touch panel or the like on which the user is able to enter various items.

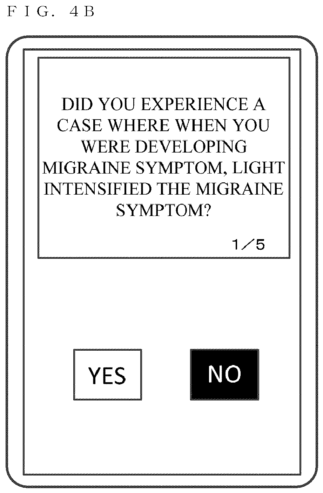

On an interface screen shown in Fig. 4A, the user is able to choose an affirmative answer "Yes" or a negative answer "No" when answering a question "Do you have a migraine?". When the user chooses "No", the interface displays a screen that recommends an ophthalmic lens that selectively cuts light with a wavelength around 460 nm, the ophthalmic lens being extracted from lens information, for a case where user information indicates that the user uses a PC (personal computer) on a daily basis. When "Yes" is chosen, as shown in Fig. 4A, the screen switches to an interface screen of Fig. 4B. It should be noted that on the drawings, a chosen item is shown as white characters in a black background.

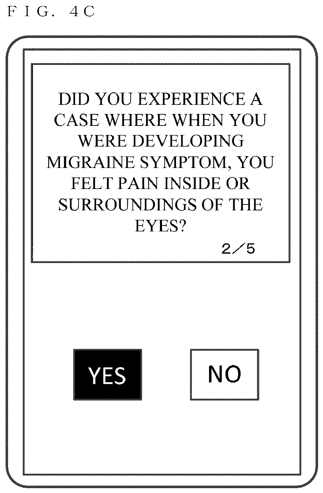

On the interface screen shown in Fig. 4B, out of the questions making up the question information, the question 1 "Did you experience a case where when you were developing a migraine symptom, light intensified the migraine symptom?" is displayed. In response to this question, the user chooses an affirmative answer "Yes" or a negative answer "No". At this time, answer information is stored in the storage unit 220. The example of Fig. 4B shows a case where "No" is chosen by the user. A case of choosing "Yes" results in switching to an interface screen of Fig. 4C in the same manner as in the case of choosing "No".

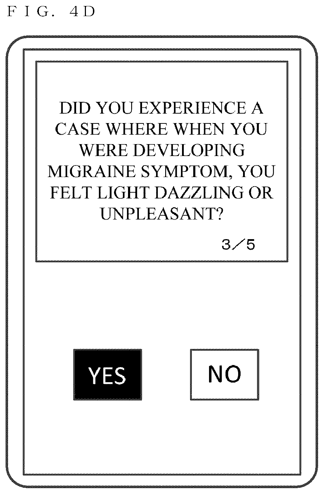

On the interface screen shown in Fig. 4C, out of the questions making up the question information, the question 2 "Did you experienced a case where when you were developing a migraine symptom, you felt a pain inside or the surroundings of the eyes?" is displayed. In response to this question, the user chooses an affirmative answer "Yes" or a negative answer "No". At this time, answer information is stored in the storage unit 220. The example of Fig. 4C shows a case where "Yes" is chosen by the user. Subsequently, the screen switches to an interface screen of Fig. 4D.

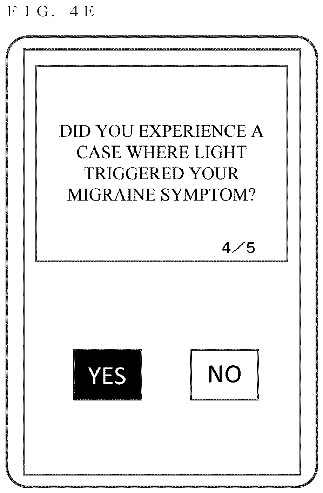

On the interface screen shown in Fig. 4D, out of the questions making up the question information, the question 3 "Did you experience a case where when you were developing a migraine symptom, you felt light dazzling or unpleasant?" is displayed. In response to this question, the user chooses an affirmative answer "Yes" or a negative answer "No". At this time, answer information is stored in the storage unit 220. The example of Fig. 4D shows a case where "Yes" is chosen by the user. Subsequently, the screen switches to an interface screen of Fig. 4E.

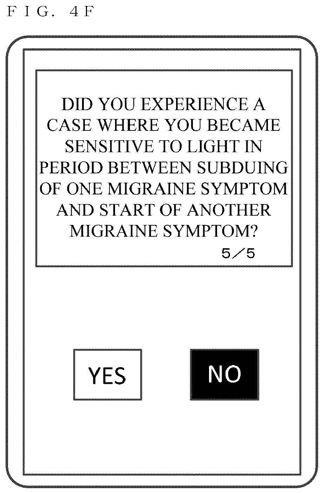

On the interface screen shown in Fig. 4E, out of the questions making up the question information, the question 4 "Did you experience a case where light triggered your migraine symptom?" is displayed. In response to this question, the user chooses an affirmative answer "Yes" or a negative answer "No". At this time, answer information is stored in the storage unit 220. The example of Fig. 4E shows a case where "Yes" is chosen by the user. Subsequently, the screen switches to an interface screen of Fig. 4F.

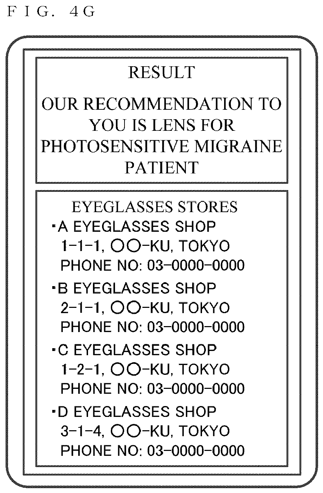

On the interface screen shown in Fig. 4F, out of the questions making up the question information, the question 5 "Did you experience a case where you became sensitive to light in a period between subduing of one migraine symptom and the start of another migraine symptom?" is displayed. In response to this question, the user chooses an affirmative answer "Yes" or a negative answer "No". At this time, answer information is stored in the storage unit 220. The example of Fig. 4F shows a case where "No" is chosen by the user. Subsequently, the screen switches to an interface screen of Fig. 4G.

On the interface screen shown in Fig. 4G, a list of candidates for stores that sell the ophthalmic lens recommended as the lens to be used for preventing or alleviating the migraine symptom of the user is displayed, based on the information on the residence of the user, together with a message "Our recommendation to you is a lens for a photosensitive migraine patient", which is a determination result given by the determination unit 230.

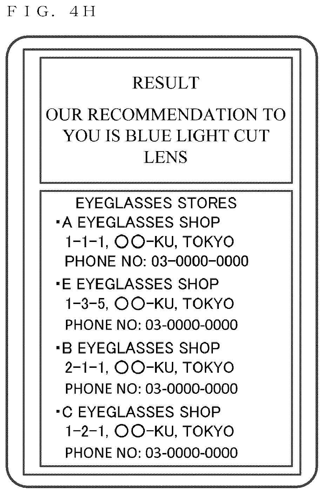

When answers to the questions 1 to 5 are all negative answers "No", the interface screen shown in Fig. 4F switches not to the interface screen shown in Fig. 4G but to an interface screen shown in Fig. 4H.

On the interface screen shown in Fig. 4H, together with a message "Our recommendation to you is a blue light cut lens", which is a message of recommending an ophthalmic lens for digital device users that selectively cuts light with a wavelength close to 460 nm, a list of candidates for stores that sell such an ophthalmic lens recommended as the lens to be used for preventing or alleviating the migraine symptom of the user is displayed, based on the information on the residence of the user.

As described above, the presence or absence of a possibility of a cause of a migraine symptom of the user being a light-based cause is determined such that the presence of a possibility of a cause of a migraine symptom of the user being a light-based cause is determined when at least one of answers to questions making up the question information shown in Fig. 3 is an affirmative answer "Yes". When "Yes" is chosen in the course of answering the questions 1 to 5, therefore, display of those questions may be terminated at the point of choosing "Yes" to switch to the interface screen of Fig. 4G.

In addition, the form of displaying the questions is not limited to the interface screens shown in Figs. 4B to 4F. For example, the questions 1 to 5 may be displayed simultaneously on a single screen.

### <Effects of First Embodiment>

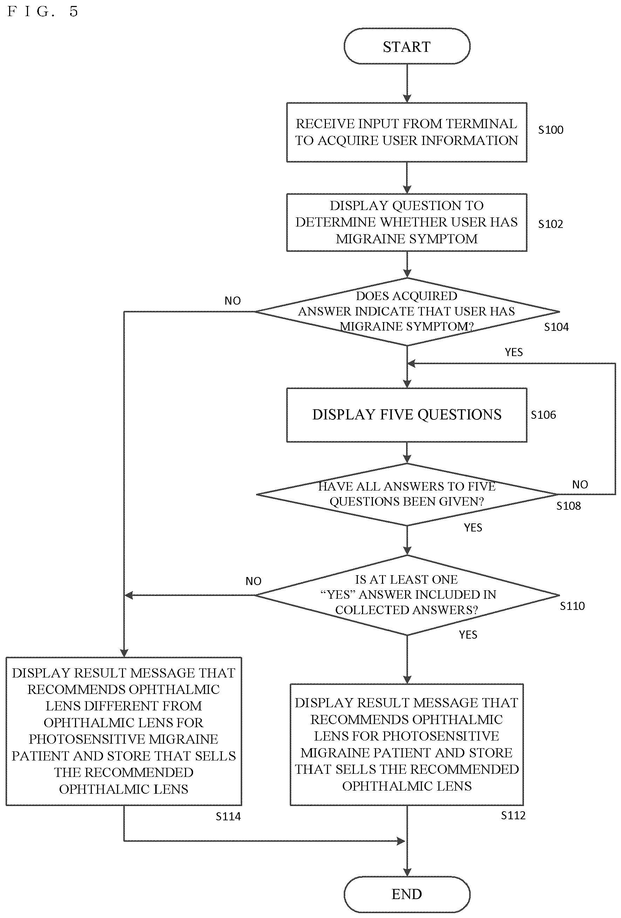

Fig. 5 depicts an example of a process routine executed by the headache cause determination apparatus 200. Effects of the headache cause determination apparatus 200 will be described with reference to Fig. 5.

At step S100, the collecting unit 210 collects user information inputted to the interface screen of the terminal 100. Following acquisition of the user information, the process routine proceeds to the next step S102.

At step S102, a question to determine whether the user has a migraine symptom is displayed on the interface screen of the terminal 100 (see Fig. 4A). The process routine then proceeds to the next step S104.

At step S104, the collecting unit 210 collects an answer to the question displayed at the above step S102, and whether the answer is "Yes", that is, whether the user has a migraine symptom is determined. When the user has a migraine symptom, the process routine proceeds to the next step S106.

At step S106, five questions are displayed respectively on the interface screens of the terminal 100 (see Figs. 4B, 4C, 4D, 4E, and 4F). The process routine then proceeds to the next step S108.

At step S108, whether all answers to five questions have been given is determined. When it is determined that all answers to five questions have been given, the process routine proceeds to the next step S110. When it is not determined that all answers to five questions have been given, the process of step S106 is continued.

At step S110, the collecting unit 210 collects answers to the questions displayed at step S106, and whether at least one "Yes" answer is included in the collected answers. When at least one "Yes" answer is included in the collected answers, the process routine proceeds to the next step S 112.

At step S112, a result that recommends an ophthalmic lens for photosensitive migraine patients and a candidate for a store that sells the ophthalmic lens, the candidate for the store being determined based on the user information, are displayed (see Fig. 4G). The process routine then comes to an end.

When it is determined at the above step S104 that the user does not have a migraine symptom, the process routine proceeds to step S114, at which, based on the user information, a result that recommends an ophthalmic lens different from an ophthalmic lens for photosensitive migraine patients and a candidate for a store that sells the recommended ophthalmic lens are displayed (see Fig. 4H). When it is not determined at step S 110 that at least one "Yes" answer is included in the collected answers, the process routine proceeds to step S114 as in the above case, at which step S114 a result that recommends an ophthalmic lens different from an ophthalmic lens for photosensitive migraine patients and a candidate for a store that sells the recommended ophthalmic lens are displayed (see Fig. 4H). The process routine then comes to an end.

As described above, according to the headache cause determination system 10 of the first embodiment, whether a cause of a migraine symptom of the user is light can be determined as answers to a plurality of questions are taken into consideration. In addition, an ophthalmic lens recommended to the user and a store that sells the ophthalmic lens can be presented.

### <System Configuration According to Second Embodiment>

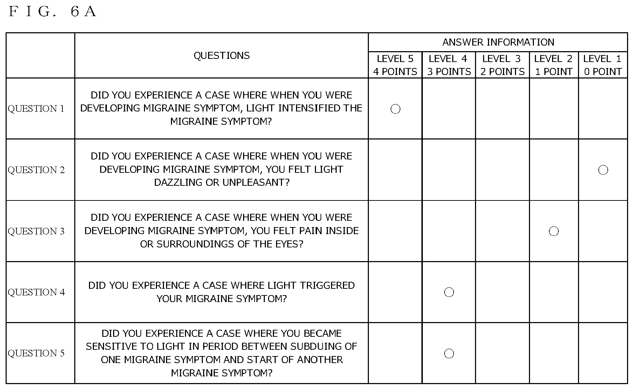

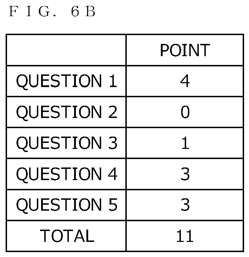

A second embodiment will be described with reference to Figs. 6A and 6B. In the first embodiment described above, when at least one of answers to questions 1 to 5 is chosen as an affirmative answer "Yes", the presence of a possibility of a cause of a migraine symptom of the user being a light-based cause is determined, and an ophthalmic lens recommended as a lens to be used for preventing or alleviating the migraine symptom of the user is determined. In the second embodiment, answers to the question information are not given by choosing "Yes" or "No" answers but by choosing levels of subjective symptoms' matching the descriptive contents of questions to score matching level points. When a matching level point exceeds a given threshold, the presence of a possibility of a cause of a migraine symptom of the user being a light-based cause is determined, and an ophthalmic lens recommended as a lens to be used for preventing or alleviating the migraine symptom of the user is determined.

The second embodiment will be described with focus placed on differences with the first embodiment, and overlapping elements will be described briefly or omitted in further description.

Fig. 6A depicts question information and answer information. Specifically, when the question information is the question 1 "Did you experience a case where when you were developing a migraine symptom, light intensified the migraine symptom?", answer candidates are displayed on the interface screen, not as "Yes" or "No" but as "level 1" to "level 5", and the user chooses one of "level 1" to "level 5", based on the user's subjective symptom. For example, "4 points" are set for "level 5", "3 points" are set for "level 4", "2 points" are set for "level 3", "1 point" is set for "level 2", and "0 point" is set for "level 1". This case is described as a case where "level 5" is chosen. In this case, therefore, "4 points" is stored in the storage unit 220, as answer information (see Fig. 6B).

An answer information column filled with "∘ (circle)" in Fig. 6A indicates an answer chosen by the user.

When the question information is the question 2 "Did you experience a case where when you were developing a migraine symptom, you felt light dazzling or unpleasant?", answer candidates are displayed on the interface screen, as "level 1" to "level 5 ", and the user chooses one of "level 1" to "level 5", based on the user's subjective symptom. For example, "4 points" are set for "level 5", "3 points" are set for "level 4", "2 points" are set for "level 3", "1 point" is set for "level 2", and "0 point" is set for "level 1". This case is described as a case where "level 1" is chosen. In this case, therefore, "0 point" is stored in the storage unit 220, as answer information (see Fig. 6B).

When the question information is the question 3 "Did you experience a case where when you were developing a migraine symptom, you felt a pain inside or the surroundings of the eyes?", answer candidates are displayed on the interface screen, as "level 1" to "level 5", and the user chooses one of "level 1" to "level 5", based on the user's subjective symptom. For example, "4 points" are set for "level 5", "3 points" are set for "level 4", "2 points" are set for "level 3", "1 point" is set for "level 2", and "0 point" is set for "level 1". This case is described as a case where "level 2" is chosen. In this case, therefore, "1 point" is stored in the storage unit 220, as answer information (see Fig. 6B).

When the question information is the question 4 "Did you experience a case where light triggered your migraine symptom?", answer candidates are displayed on the interface screen, as "level 1" to "level 5", and the user chooses one of "level 1" to "level 5", based on the user's subjective symptom. For example, "4 points" are set for "level 5", "3 points" are set for "level 4", "2 points" are set for "level 3", "1 point" is set for "level 2", and "0 point" is set for "level 1". This case is described as a case where "level 4" is chosen. In this case, therefore, "3 point" is stored in the storage unit 220, as answer information (see Fig. 6B).

When the question information is the question 5 "Did you experience a case where you became sensitive to light in a period between subduing of one migraine symptom and the start of another migraine symptom?", answer candidates are displayed on the interface screen, as "level 1" to "level 5", and the user chooses one of "level 1" to "level 5", based on the user's subjective symptom. For example, "4 points" are set for "level 5", "3 points" are set for "level 4", "2 points" are set for "level 3", "1 point" is set for "level 2", and "0 point" is set for "level 1". This case is described as a case where "level 4" is chosen. In this case, therefore, "3 point" is stored in the storage unit 220, as answer information (see Fig. 6B).

The determination unit 230 counts points of answer information stored in the storage unit 220 to produce a total point. In this example, the total point is "11", as shown in Fig. 6B. Then, whether the total point is equal to or larger than a given threshold value of, for example, "10 points" is determined. In this example, because the total point, i.e., "11 points" is larger than "10 points", the presence of a possibility of a cause of a migraine symptom of the user being a light-based cause is determined, and an ophthalmic lens recommended as a lens to be used for preventing or alleviating the migraine symptom of the user is determined.

For all of the questions 1 to 5, the points are set respectively for the levels at the same proportion. Point/level setting is, however, not limited to this case. For example, for the questions 1 to 3, "10 points" and "9 points" may be set respectively for "level 5" and "level 4", which means that a proportion of point allocation for the questions 1 to 3 is higher than that for the questions 4 and 5.

The given threshold may be determined to be a point other than "10 points", or may be increased or decreased according to the user information. It is a known fact that women in their 20's to 40's are likely to develop migraine symptoms, and for such users, the given threshold may be determined to be lower than that for other users.

The determination unit 230 counts the points, based on the answer information, and determines the presence of a possibility of a cause of a migraine symptom of the user being a light-based cause. The determination unit 230, however, may make its determination by a different approach. For example, the determination unit 230 may input answer information to a learned model created by incorporating learning data composed of the weight of answer information and correct data on other users in the past, thereby determining the presence of a possibility of a cause of a migraine symptom of the user being a light-based cause.

As described above, according to the headache cause determination system 10 of the second embodiment, whether a cause of a migraine symptom of the user is light can be determined as answers to a plurality of questions are taken into consideration. In addition, an ophthalmic lens recommended to the user and a store that sells the ophthalmic lens can be presented.

### <System Configuration According to Third Embodiment>

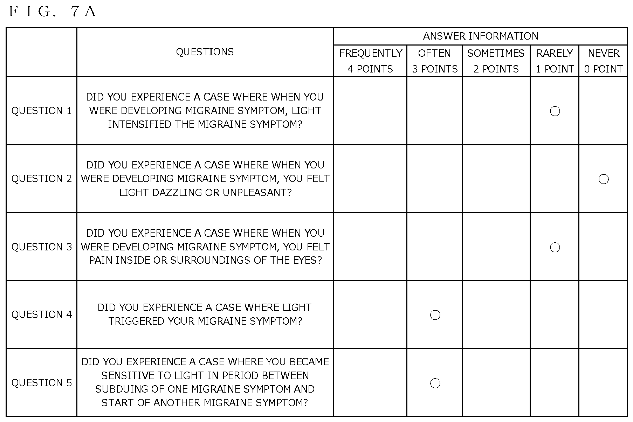

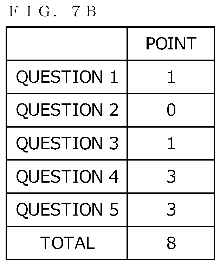

A third embodiment will be described with reference to Figs. 7A and 7B. In the first embodiment described above, when at least one of answers to questions 1 to 5 is chosen as an affirmative answer "Yes", the presence of a possibility of a cause of a migraine symptom of the user being a light-based cause is determined, and an ophthalmic lens recommended as a lens to be used for preventing or alleviating the migraine symptom of the user is determined. In the third embodiment, answers to the question information are not given by choosing "Yes" or "No" answers but by choosing frequencies of occurrence of subjective symptoms for respective questions to score frequency points. When a frequency point exceeds a given threshold, the presence of a possibility of a cause of a migraine symptom of the user being a light-based cause is determined, and an ophthalmic lens recommended as a lens to be used for preventing or alleviating the migraine symptom of the user is determined.

The third embodiment will be described with focus placed on differences with the first embodiment, and overlapping elements will be described briefly or omitted in further description.

Fig. 7A depicts question information and answer information. Specifically, when the question information is the question 1 "Did you experience a case where when you were developing a migraine symptom, light intensified the migraine symptom?", answer candidates are displayed on the interface screen, not as "Yes " or" No " but as "Frequently", "Often", "Sometimes", "Rarely", and "Never", and the user chooses one of "Frequently", "Often", "Sometimes", "Rarely", and "Never", based on the user's subjective symptom. For example, "4 points" are set for "Frequently", "3 points" are set for "Often", "2 points" are set for "Sometimes", "1 point" is set for "Rarely", and "0 point" is set for "level 1". This case is described as a case where "Rarely" is chosen. In this case, therefore, "1 point" is stored in the storage unit 220, as answer information (see Fig. 7B).

An answer information column filled with "∘ (circle)" in Fig. 7A indicates an answer chosen by the user.

When the question information is the question 2 "Did you experience a case where when you were developing a migraine symptom, you felt light dazzling or unpleasant?", answer candidates are displayed on the interface screen, as "Frequently", "Often", "Sometimes", "Rarely", and "Never", and the user chooses one of "Frequently", "Often", "Sometimes", "Rarely", and "Never", based on the user's subjective symptom. For example, "4 points" are set for "Frequently", "3 points" are set for "Often", "2 points" are set for "Sometimes", "1 point" is set for "Rarely", and "0 point" is set for "level 1". This case is described as a case where "Never" is chosen. In this case, therefore, "0 point" is stored in the storage unit 220, as answer information (see Fig. 7B).

When the question information is the question 3 "Did you experience a case where when you were developing a migraine symptom, you felt a pain inside or the surroundings of the eyes?", answer candidates are displayed on the interface screen, as "Frequently", "Often", "Sometimes", "Rarely", and "Never", and the user chooses one of "Frequently", "Often", "Sometimes", "Rarely", and "Never", based on the user's subjective symptom. For example, "4 points" are set for "Frequently", "3 points" are set for "Often", "2 points" are set for "Sometimes", "1 point" is set for "Rarely", and "0 point" is set for "level 1". This case is described as a case where "Rarely" is chosen. In this case, therefore, "1 point" is stored in the storage unit 220, as answer information (see Fig. 7B).

When the question information is the question 4 "Did you experience a case where light triggered your migraine symptom?", answer candidates are displayed on the interface screen, as "Frequently", "Often", "Sometimes", "Rarely", and "Never", and the user chooses one of "Frequently", "Often", "Sometimes", "Rarely", and "Never", based on the user's subjective symptom. For example, "4 points" are set for "Frequently", "3 points" are set for "Often", "2 points" are set for "Sometimes", "1 point" is set for "Rarely", and "0 point" is set for "level 1". This case is described as a case where "Sometimes" is chosen. In this case, therefore, "3 points" is stored in the storage unit 220, as answer information (see Fig. 7B).

When the question information is the question 5 "Did you experience a case where you became sensitive to light in a period between subduing of one migraine symptom and the start of another migraine symptom?", answer candidates are displayed on the interface screen, as "Frequently", "Often", "Sometimes", "Rarely", and "Never", and the user chooses one of "Frequently", "Often", "Sometimes", "Rarely", and "Never", based on the user's subjective symptom. For example, "4 points" are set for "Frequently", "3 points" are set for "Often", "2 points" are set for "Sometimes", "1 point" is set for "Rarely", and "0 point" is set for "level 1". This case is described as a case where "Sometimes" is chosen. In this case, therefore, "3 points" is stored in the storage unit 220, as answer information (see Fig. 7B).

The determination unit 230 counts points of answer information stored in the storage unit 220 to produce a total point. In this example, the total point is "8", as shown in Fig. 7B. Then, whether the total point is equal to or larger than the given threshold value of, for example, "10 points" is determined. In this example, because the total point, i.e., "8 points" is neither equal to nor larger than "10 points", the presence of a possibility of a cause of a migraine symptom of the user being a light-based cause is not determined. For this reason, it is determined that not an ophthalmic lens recommended as a lens to be used for preventing or alleviating the migraine symptom of the user but an ophthalmic lens for digital device users that selectively cuts light with a wavelength close to 460 nm is recommended.

For all of the questions 1 to 5, the points are set respectively for the frequencies of occurrence of subjective symptoms at the same proportion. Point/frequency setting is, however, not limited to this case. For example, for the questions 1 to 3, "10 points" and "9 points" may be set respectively for "Frequently" and "Often", which means that a proportion of point allocation for the questions 1 to 3 is higher than that for the questions 4 and 5.

The given threshold may be determined to be a point other than "10 points", or may be increased or decreased according to the user information. It is a known fact that women in their 20's to 40's are likely to develop migraine symptoms, and for such users, the given threshold may be determined to be lower than that for other users.

The determination unit 230 counts the points, based on the answer information, and determines the presence of a possibility of a cause of a migraine symptom of the user being a light-based cause. The determination unit 230, however, may make its determination by a different approach. For example, the determination unit 230 may input answer information to a learned model created by incorporating learning data composed of the weight of answer information and correct data on other users in the past, thereby determining the presence of a possibility of a cause of a migraine symptom of the user being a light-based cause.

As described above, according to the headache cause determination system 10 of the third embodiment, whether a cause of a migraine symptom of the user is light can be determined as answers to a plurality of questions are taken into consideration. In addition, an ophthalmic lens recommended to the user and a store that sells the ophthalmic lens can be presented.

### <System Configuration According to Fourth Embodiment>

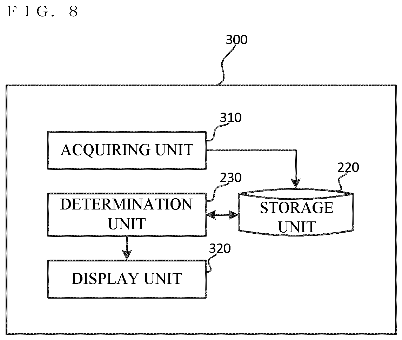

A fourth embodiment will be described with reference to Fig. 8.

Fig. 8 is a block diagram showing a system configuration of a headache cause determination apparatus 300 according to a fourth embodiment. The headache cause determination apparatus 300 having the configuration shown in Fig. 8 includes an acquiring unit 310, the storage unit 220, the determination unit 230, and a display unit 320.

In the above first embodiment, the collecting unit 210 collects user information and answer information transmitted from a plurality of terminals, and the determination unit 230 outputs its determination result to the terminals. In the fourth embodiment, the acquiring unit 310 acquires user information and answer information, and the display unit 320 displays a determination result given by the determination unit 230.

The fourth embodiment will be described with focus placed on differences with the first to third embodiments, and overlapping elements will be described briefly or omitted in further description.

The acquiring unit 310 of the headache cause determination apparatus shown in Fig. 8 acquires user information and answer information inputted on an input device, such as a touch panel (see Figs. 4A to 4H), connected to the headache cause determination apparatus 300, and stores the user information and answer information in the storage unit 220.

The storage unit 220 and the determination unit 230 of the headache cause determination apparatus 300 shown in Fig. 8 have the same functions as those of the storage unit 220 and the determination unit 230 of the first embodiment.

The display unit 320 of the headache cause determination apparatus 300 shown in Fig. 8 is, for example, a liquid crystal display, and displays various pieces of information under control by the CPU. In the fourth embodiment, the display unit 320 displays information on ophthalmic lenses and candidates for stores that sell the ophthalmic lenses, as a determination result given by the determination unit 230. The display unit 320 may be provided as a touch panel to function as an input device.

As described above, according to the headache cause determination system 10 of the fourth embodiment, whether a cause of a migraine symptom of the user is light can be determined as answers to a plurality of questions are taken into consideration. In addition, an ophthalmic lens recommended to the user and a store that sells the ophthalmic lens can be presented.

It should be noted that the present invention is not limited to the above embodiments, and various modifications and applications of the invention may be made within a range that does not depart from the substance of the invention.

For example, the embodiments have been described herein as the embodiment in which a program is installed in advance. Such a program may be provided as a program stored in a computer-readable recording medium.

Another modification may also be possible, in which a doctor or a sales clerk at a store that sells ophthalmic lenses outputs question information on a headache symptom of the user, acquires answer information on answers to the question information, and based on the acquired answer information, determines the presence or absence of a possibility of a cause of a migraine symptom of the user being a light-based cause.

The disclosure of Japanese Patent Application No. 2020-123104 filed on July 17, 2020 is entirely incorporated herein by reference.

All documents, patent applications, and technical standards described in this specification are incorporated herein by reference to the same extent as the extent to which individual documents, patent applications, and technical standards are incorporated by reference in individual cases where reference-based incorporation of documents, patent applications, and technical standards is stated specifically.

## Claims

1. A headache cause determination system that is configured to determine whether or not there is a possibility that a cause of a migraine symptom, of a user suffering from the migraine symptom, is a light-based cause, the system comprising a computer, wherein:
the computer includes a CPU, a storage unit, and a determination unit executed by the CPU,
the storage unit is configured to store data that enables a determination as to whether or not there is a possibility that a cause of the migraine symptom of the user is a light-based cause, based on question information regarding the migraine symptom of the user and response information regarding the user's response to the question information, and
the determination unit is configured to determine whether or not there is a possibility that a cause of the migraine symptom of the user is a light-based cause, based on the response information regarding the user's response to the question information and based on the data stored in the storage unit, and to output a result of the determination.

2. The headache cause determination system according to claim 1, wherein:
when the determination unit has determined that there is a possibility that a cause of a migraine symptom of the user is a light-based cause, the storage unit further stores lens information regarding an ophthalmic lens recommended as a lens to be used for preventing or alleviating the migraine symptom, and
when the determination unit has determined that there is a possibility that a cause of the migraine symptom of the user is a light-based cause, the determination unit outputs a result of the determination, and, from the lens information, selects and outputs the ophthalmic lens recommended as the lens to be used for preventing or alleviating the migraine symptom of the user.

3. The headache cause determination system according to claim 2, wherein:
the storage unit further stores user information including information regarding a residence of the user, and retail store information regarding a retail store that sells an ophthalmic lens recommended as a lens to be used for preventing or alleviating a migraine symptom of the user, and
when selecting and outputting an ophthalmic lens recommended as a lens to be used for preventing or alleviating the migraine symptom of the user, the determination unit selects and outputs a candidate for a retail store that sells the ophthalmic lens recommended as the lens to be used for preventing or alleviating the migraine symptom of the user, based on the information regarding the residence of the user and the retail store information.

4. The headache cause determination system according to any one of claim 1 to claim 3, wherein the question information comprises a question about a condition of the user who is developing the migraine symptom, a question about a cause that triggers the migraine symptom, or a question about a condition of the user before and after the user develops the migraine symptom.

5. The headache cause determination system according to claim 4, wherein the question about a condition of the user who is developing the migraine symptom is presented in order to ascertain whether light intensifies a migraine symptom of the user when the user is developing the migraine symptom or whether the user feels light to be dazzling or unpleasant when the user is developing the migraine symptom.

6. The headache cause determination system according to claim 5, wherein the question about a condition of the user who is developing the migraine symptom is presented in order to ascertain whether light causes the user any pain inside or surrounding their eyes when the user is developing migraine symptom.

7. The headache cause determination system according to any one of claim 4 to claim 6, wherein the question about a cause that triggers the migraine symptom of the user is presented in order to ascertain whether the user has experienced a case in which light has triggered the migraine symptom.

8. The headache cause determination system according to any one of claim 4 to claim 7, wherein the question about the condition of the user before and after the user develops the migraine symptom is presented in order to ascertain whether the user has experienced a case in which the user has become sensitive to light in a period between one migraine symptom subsiding and another migraine symptom starting.

9. A headache cause determination method that causes a computer to execute processing, the processing comprising:
determining whether or not there is a possibility that a cause of a migraine symptom of a user is a light-based cause, based on data that enables a determination as to whether or not there is a possibility that a cause of a migraine symptom of a user is a light-based cause, the determination being based on question information regarding the migraine symptom of the user and response information regarding the user's response to the question information, the data being stored in a storage unit of the computer; and
outputting a result of the determination.

10. The headache cause determination method according to claim 9, the method causing a computer to execute processing comprising:
causing the storage unit to further store user information including information regarding a residence of the user and retail store information regarding a retail store that sells an ophthalmic lens recommended as a lens to be used for preventing or alleviating a migraine symptom of the user; and
when selecting and outputting an ophthalmic lens recommended as a lens to be used for preventing or alleviating the migraine symptom of the user, selecting and outputting a candidate for a retail store that sells the ophthalmic lens recommended as the lens to be used for preventing or alleviating the migraine symptom of the user, based on the information regarding the residence of the user and the retail store information.

11. The headache cause determination method according to claim 9 or claim 10, wherein the question information comprises a question about a condition of the user who is developing a migraine symptom, a question about a cause that triggers a migraine symptom of the user, or a question about a condition of the user before and after the user develops a migraine symptom.

12. A program that, in a headache cause determination system including a storage unit and a determination unit, is executable by a computer to function as the determination unit, the determination unit being configured to:
determine whether or not there is a possibility that a cause of a migraine symptom of a user is a light-based cause, based on data that enables a determination as to whether or not there is a possibility that a cause of a migraine symptom of a user is a light-based cause, the determination being based on question information regarding the migraine symptom of the user and response information regarding the user's response to the question information, the data being stored in a storage unit of the computer; and
output a result of the determination.

13. The program according to claim 12, wherein:
the program causes the storage unit to further store user information including information regarding a residence of the user and retail store information regarding a retail store that sells an ophthalmic lens recommended as a lens to be used for preventing or alleviating a migraine symptom of the user, and
when the determination unit selects and outputs an ophthalmic lens recommended as a lens to be used for preventing or alleviating the migraine symptom of the user, the program causes the determination unit to select and output a candidate for a store that sells the ophthalmic lens recommended as the lens to be used for preventing or alleviating the migraine symptom of the user, based on the information regarding the residence of the user and the retail store information.

14. The program according to claim 12 or claim 13, wherein the question information comprises a question about a condition of the user who is developing a migraine symptom, a question about a cause that triggers a migraine symptom of the user, or a question about a condition of the user before and after the user develops a migraine symptom.

15. A data structure, comprising:
question information regarding a migraine symptom of a user;
response information regarding the user's response to the question information; and
data that enables a determination as to whether or not there is a possibility that a cause of the migraine symptom of the user is a light-based cause, based on the question information and the response information,
wherein the data structure causes a computer to determine whether or not there is a possibility that a cause of the migraine symptom of the user is a light-based cause, based on the response information regarding the user's response to the question information and on the data, and to output a result of the determination.

16. The data structure according to claim 15, further comprising:
user information including information regarding a residence of the user; and
retail store information regarding a retail store that sells an ophthalmic lens recommended as a lens to be used for preventing or alleviating a migraine symptom of the user,
wherein, when an ophthalmic lens recommended as a lens to be used for preventing or alleviating the migraine symptom of the user has been selected and output, the data structure causes a computer to select and output a candidate for a store that sells the ophthalmic lens recommended as the lens to be used for preventing or alleviating the migraine symptom of the user, based on the information regarding the residence of the user and the retail store information.

17. The data structure according to claim 15 or claim 16, wherein the question information comprises a question about a condition of the user who is developing a migraine symptom, a question about a cause that triggers a migraine symptom of the user, or a question about a condition of the user before and after the user develops a migraine symptom.
